# EUROPÄISCHE PATENTSCHRIFT

(11) **EP 0 781 762 B1**
(45) Veröffentlichungstag und Bekanntmachung des Hinweises auf die Patenterteilung: **07.01.2004**
(21) Anmeldenummer: 97101598.7
(22) Anmeldetag: 07.06.1994
(51) Int. Cl.: C07C 229/16, C07C 227/00, C07C 229/30, C07C 255/25

(54) **Verfahren zur Herstellung von Glycin-N.N.-diesigsäure-Derivaten**
Process for preparing glycine-N,N-diacetic acid derivatives
Procédé pour la préparation de dérivés de glycine-N,N-acide diacétique

(30) Priorität: 16.06.1993 DE 4319935
(43) Veröffentlichungstag der Anmeldung: 02.07.1997
(62) Teilanmeldung aus: 94920434.1
(73) Patentinhaber: BASF AKTIENGESELLSCHAFT, 67056 Ludwigshafen (DE)
(72) Erfinder: Schneider, Jürgen, Dr., 67251 Freinsheim (DE); Potthoff-Karl, Birgit, Dr., 67061 Ludwigshafen (DE); Kud, Alexander, Dr., 55234 Eppelsheim (DE); Baur, Richard, Dr., 67112 Mutterstadt (DE); Oftring, Alfred, Dr., 67098 Bad Dürkheim (DE); Greindl, Thomas, Dr., 94127 Neuburg (DE)

(56) Entgegenhaltungen:
- DE-A- 1 643 415
- DE-A- 2 027 972
- DE-A- 3 712 329
- DE-A- 4 211 713
- CHEMICAL ABSTRACTS, vol. 106, no. 18, 4. Mai 1987 (1987-05-04) Columbus, Ohio, US; abstract no. 140140c, S. LUEBKEOVA ET AL : "Surfactants from monoamine complexons." Seite 103; XP002902849 & CZECH. CS 199,072,

## Beschreibung

Die vorliegende Erfindung betrifft ein Verfahren zur Herstellung von Glycin-N,N-diessigsäure-Derivaten und bei diesem Verfahren auftretende Zwischenprodukte.

Die DE-A 37 12 329 offenbart ein Verfahren zur Herstellung von Serin-N,N-diessigsäure-Derivaten durch Umsetzung eines hydroxymethylsubstituierten Glycins mit Formaldehyd und Cyanwasserstoff.

Aufgabe der vorliegenden Erfindung war es, ein Verfahren zur Herstellung von Glycin-N,N-diessigsäure-Derivaten bereitzustellen, welche die Nachteile des Standes der Technik nicht mehr aufweisen.

Demgemäß wurde ein Verfahren zur Herstellung von Glycin-N,N-diessigsäure-Derivaten der allgemeinen Formel I 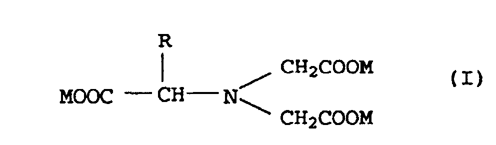 in der
- R: für Methyl, Ethyl, n-Propyl oder iso-Butyl steht und
- M: Wasserstoff, Alkalimetall, Erdalkalimetall, Ammonium oder substituiertes Ammonium in den entsprechenden stöchiometrischen Mengen bedeutet,
gefunden, welches dadurch gekennzeichnet ist, daß man Iminodiessigsäure oder Iminodiacetonitril mit entsprechenden Monoaldehyden und Cyanwasserstoff oder Alkalimetallcyanid umsetzt und anschließend noch vorhandene Nitrilgruppen zu Carboxylgruppe hydrolysiert.

Die genannte Ausführungsform stellt ein Beispiel für die "Strekker-Synthese" dar, bei der allgemein Aldehyde mit Ammoniak oder Aminen und Blausäure ("saure" Variante) oder Cyaniden ("alikalische" Variante) zu Aminosäuren oder Derivate hiervon umgesetzt werden.

Die "alkalische" Variante der Strecker-Synthese wird beispielsweise in der US-A 3 733 355 in allgemeiner Form dargestellt. Die dort aufgeführten Beispiele zeigen jedoch, daß immer ein hoher Anteil an Nebenprodukten, vor allem an unerwünschter Glykolsäure, auftritt; dies läßt sich aus den Umsätzen von nur maximal ca. 89 % schließen.

Die "saure" Variante der Strecker-Synthese ist beispielsweise aus der DE-A 20 27 972 bekannt. Dort wird die Herstellung von Carboxymethyliminodiacetonitril ausgehend von Glycin, Formaldehyd und Blausäure in saurem Medium beschrieben. Hierbei wird empfohlen, zusätzliche Säure zuzugeben, um den pH-Wert im Bereich unterhalb von 7 zu halten.

Aufgabe der vorliegenden Erfindung war es auch, ein effektiveres und wirtschaftlicheres Verfahren zur Herstellung von Glycin-N,N-diessigsäure-Derivaten I bereitzustellen, das insbesondere die Bildung von unerwünschten Nebenprodukten unterdrückt und auf zusätzliche Hilfsstoffe, beispielsweise zur pH-Wert-Regulierung, verzichten kann.

Als besonders vorteilhaft haben sich die Varianten herausgestellt, bei denen mit Cyanwasserstoff ("saure" Variante) gearbeitet wird. Zweckmäßigerweise setzt man wasserfreien Cyanwasserstoff ein, der großtechnisch üblicherweise in dieser Form gehandhabt wird.

Die Umsetzung wird bevorzugt in Wasser, aber auch in einem organischen Lösungsmittel oder in Mischungen hieraus durchgeführt. Als organische Lösungsmittel kommen vorzugsweise solche zum Einsatz, die mit Wasser teilweise oder vollständig mischbar sind, z. B. Methanol, Ethanol, n-Propanol, iso-Propanol, tert.-Butanol, Dioxan oder Tetrahydrofuran. Auch Lösungsvermittler können verwendet werden.

Man setzt zweckmäßigerweise pro Mol Aminoverbindung 2 bis 2,6 mol Aldehyd, in wasserfreier Form oder als wäßrige Lösung, und 2 bis 2,3 mol Cyanwasserstoff oder Alkalimetallcyanid, beispielsweise Natrium- oder Kaliumcyanid, ein. Die Umsetzung wird normalerweise im Falle von wasserfreiem Cyanwasserstoff bei Temperaturen von 0 bis 120°C, insbesondere 15 bis 80°C, und im Falle von Alkalimetallcyaniden bei 40 bis 110°C, insbesondere 70 bis 100°C, vorgenommen. Für die Umsetzung mit wasserfreiem Cyanwasserstoff kommt bei Mitverwendung von Mineralsäuren wie Schwefel-, Salz- oder ortho-Phosphorsäure in der Regel ein pH-Bereich von 0 bis 11, insbesondere von 1 bis 9, in Betracht, bei der Umsetzung mit Alkalimetallcyaniden arbeitet man üblicherweise bei pH 10 bis 14, insbesondere 11 bis 13.

An diese Umsetzung schließt sich eine Hydrolyse noch vorhandener Nitrilgruppen zu Carboxylgruppen an, welche in an sich bekannter Weise in wäßrigem Reaktionsmedium in Gegenwart von Basen wie Natron- oder Kalilauge oder von Säuren wie Schwefel- oder Salzsäure bei Temperaturen von 20 bis 110°C, insbesondere 40 bis 100°C, durchgeführt wird.

Entsprechend den Reaktionsbedingungen erhält man die Glycin-N,N-diessigsäure-Derivate I als freie Carbonsäure oder beispielsweise als Alkalimetallsalz. Aus der freien Säure können durch Neutralisation mit den entsprechenden Basen, beispielsweise Aminbasen, die gewünschten Salze ohne Schwierigkeit hergestellt werden.

Die Glycin-N,N-diessigsäure-Derivate I und ihre Salze lassen sich aus ihren Lösungen problemlos in reiner Form isolieren. Hierfür bieten sich insbesondere Sprüh- oder Gefriertrocknung, Kristallisation und Fällung an. Es kann vorteilhaft sein, die bei der Herstellung angefallene Lösung direkt einer technischen Verwendung zuzuführen.

Gegenstand der vorliegenden Erfindung sind auch die in der Literatur noch nicht bekannten in 2-Position durch den Rest R substituierten Glycin-N,N-diacetonitrile und Glycinnitril-N,N-diacetonitrile, bei denen R für Methyl, Ethyl, n-Propyl oder iso-Butyl steht, beispielsweise die Verbindungen α-Alanin-N,N-diacetonitrilund α-Alaninnitril-N,N-diacetonitril, als Zwischenprodukte für die Herstellung von Glycin-N,N-diessigsäure-Derivaten I und ihren Salzen. Diese Verbindungen entstehen als Zwischenstufen bei der Umsetzung von Iminodiacetonitril mit entsprechenden Monoaldehyden und Cyanwasserstoff.

Generell erhält man das Umsetzungsprodukt in hoher Ausbeute in ausreichend reiner Form. Der Gehalt an Nebenprodukten ist gering. Weitere Vorteile des erfindungsgemäßen Herstellungsverfahrens sind die salzfreie Fahrweise und die leicht verfügbaren Einsatzstoffe.

### Herstellungsbeispiele

### Beispiel 1

### Herstellung von α-D,L-Alanin-N,N-diessigsäure-Trinatriumsalz aus Iminodiacetonitril

Zu einer Suspension von 95 g Iminodiacetonitril (100 gew.-%ig) in 500 ml Wasser wurden nacheinander bei 35 bis 50°C 14 g Schwefelsäure (100 gew.-%ig), 27 g wasserfreie Blausäure und 44 g Acetaldehyd (100 gew.-%ig) gegeben. Man rührte solange, bis bei der Titration des Blausäuregehaltes keine Änderung mehr festzustellen war. Nach Abkühlen auf 10°C wurde der Niederschlag abfiltriert und getrocknet. Es resultierten 123,4 g α-D,L-Alaninnitril-N,N-diacetonitril (entsprechend 83 % der Theorie) vom Schmelzpunkt 82°C.

Das erhaltene α-D,L-Alaninnitril-N,N-diacetonitril wurde bei 50°C in 440 g 25 gew.-%iger wäßriger Natronlauge eingetragen, es wurde anschließend noch 2 Stunden bei dieser Temperatur nachgerührt. Danach wurde für 10 Stunden auf 95°C erwärmt. Gegen Ende der Umsetzung wurde das Reaktionsgemisch mit Wasser verdünnt. Man erhielt so 610 g einer wäßrigen Lösung von α-D,L-Alanin-N,N-diessigsäure-Trinatriumsalz mit einem Eisen-Bindevermögen von 1,285 mmol/g (entsprechend 94 % der Theorie, bez. auf eingesetztes α-D,L-Alaninnitril-N,N-diacetonitril).

### Beispiel 4

### D,L-Ethylglycin-N,N-diessigsäure-Trinatriumsalz aus Iminodiacetonitril

In eine Suspension von 570 g Iminodiacetonitril in 2070 g Wasser wurden nacheinander 41 g Schwefelsäure (96 gew.-%), 180 g Cyanwasserstoff (99 Gew.-%) und 385 g Propionaldehyd (99,5 Gew.-%) zugetropft und solange bei 35°C gerührt, bis durch Titration keine Änderung des Blausäuregehaltes mehr feststellbar war. Nach Abkühlen auf 10°C wurden 977 g (97 % Ausbeute der Theorie) D,L-Ethylglycinnitril-N,N-diacetonitril als Niederschlag durch Filtration mit einer Reinheit von 96,8 Gew.-% erhalten.

Der Niederschlag wurde dann in 4430 g einer 17 gew.-%igen wäßrigen Natronlauge bei 60°C eingetragen und 3 h bei 60°C und 10 h bei 95°C nachgerührt und am Ende mit Wasser verdünnt. Man erhielt dadurch 5275 g einer Lösung von D,L-Ethylglycon-N,N-diessigsäure-Trinatriumsalz mit einem Eisenbindevermögen von 0,985 mmol/g (entsprechend 89 % Ausbeute der Theorie).

### Beispiel 3

### D,L-Propylglycin-N,N-diessigsäure-Trinatriumsalz aus Iminodiacetonitril

In eine Suspension von 95 g Iminodiacetonitril in 550 g Wasser wurden nacheinander 14 g Schwefelsäure (96 Gew.-%), 26,9 g Cyanwasserstoff (99,3 %) und 79,3 g Butyraldehyd zugetropft und 4 h bei 35°C gerührt, bis durch Titration keine Änderung des Blausäuregehaltes mehr feststellbar war. Nach Abkühlen auf 10°C wurden 165 g (94 % Ausbeute der Theorie) D,L-n-Propylglycinnitril-N,N-diacetonitril durch Phasentrennung erhalten.

Von diesem Öl wurden dann 70,4 g in 350 g einer 15 gew.-%igen wäßrigen Natronlauge bei 40°C eingetragen und 2 h bei 95°C nachgerührt und anschließend mit Wasser verdünnt. Man erhielt dadurch 600 g einer Lösung von D,L-n-Propylglycin-N,N-diessigsäure-Trinatriumsalz mit einem Eisenbindevermögen von 0,573 mmol/g (entsprechend 86 % Ausbeute der Theorie).

### Beispiel 4

D,L-2-Methylpropylglycin-N,N-diessigsäure-Trinatriumsalz aus Iminodiacetonitril

In eine Suspension von 95 g Iminodiacetonitril in 350 g Wasser wurden nacheinander 7 g Schwefelsäure (96 Gew.-%), 30 g Cyanwasserstoff (98,3 Gew.-%) und 103,4 g 3-Methylbutyraldehyd zugetropft und 2 h bei 35°C und 3 h bei 50°C gerührt, bis durch Titration keine Änderung des Blausäuregehaltes mehr feststellbar war. Nach Abkühlen auf 10°C wurden 175 g (92 % Ausbeute der Theorie) D,L-2-Methylpropylglycinnitril-N,N-diacetonitril durch Phasentrennung erhalten.

Das erhaltene Öl wurde dann in 860 g einer 14 gew.-%igen wäßrigen Natronlauge bei 40°C eingetragen und 3 h bei 60°C und 5 h bei 95°C nachgerührt. Man erhielt dadurch 1070 g einer Lösung von D,L-2-Methylpropylglycin-N,N-diessigsäure-Trinatriumsalz mit einem Eisenbindevermögen von 0,775 mmol/g (entsprechend 90 % Ausbeute der Theorie).

## Patentansprüche

1. Verfahren zur Herstellung von Glycin-N,N-diessigsäure-Derivaten der allgemeinen Formel I 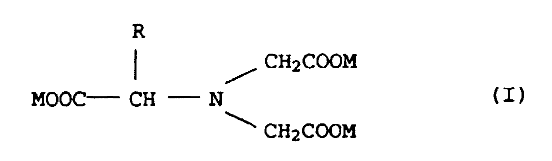 in der
R für Methyl, Ethyl, n-Propyl oder iso-Butyl steht und
M Wasserstoff, Alkalimetall, Erdalkalimetall, Ammonium oder substituiertes Ammonium in den entsprechenden stöchiometrischen Mengen bedeutet,
**dadurch gekennzeichnet, daß** man Iminodiessigsäure oder Iminodiacetonitril mit entsprechenden Monoaldehyden und Cyanwasserstoff oder Alkalimetallcyanid umsetzt und anschließend noch vorhandene Nitrilgruppen zu Carboxylgruppen hydrolysiert.

2. Verfahren zur Herstellung von α-Alanin-N,N-diessigsäure und ihren Alkalimetall-, Ammonium- und substituierten Ammoniumsalzen nach Anspruch 1.

3. Verfahren zur Herstellung von Glycin-N,N-diessigsäure-Derivaten I nach Anspruch 1, **dadurch gekennzeichnet, daß** man im pH-Bereich von 0 bis 11 arbeitet.

4. In 2-Position durch den Rest R substituierte Glycin-N,N-diacetonitrile und Glycinnitril-N,N-diacetonitrile, bei denen R für Methyl, Ethyl, n-Propyl oder iso-Butyl steht, als Zwischenprodukte.

## Claims

1. A process for the preparation of glycine-N,N-diacetic acid derivatives of the formula I 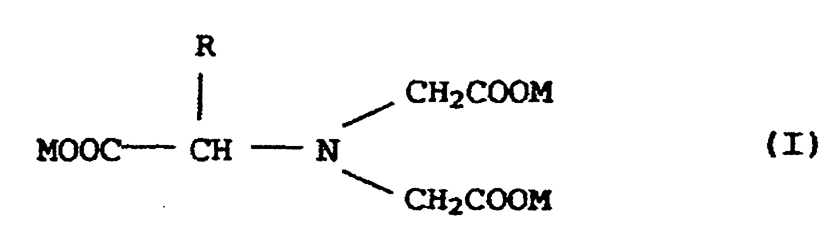 in which
R is methyl, ethyl, n-propyl or isobutyl and
M is hydrogen, alkali metal, alkaline earth metal, ammonium or substituted ammonium in the corresponding stoichiometric amounts,
which comprises reacting iminodiacetic acid or iminodiacetonitrile with corresponding monoaldehydes and hydrogen cyanide or alkali metal cyanide, and then hydrolyzing nitrile groups which are still present to carboxyl groups.

2. A process for the preparation of α-alanine-N,N-diacetic acid and its alkali metal, ammonium and substituted ammonium salts as claimed in claim 1.

3. A process for the preparation of glycine-N,N-diacetic acid derivatives I as claimed in claim 1, which comprises working in the pH range from 0 to 11.

4. Glycine-N,N-diacetonitriles and glycinonitrile-N,N-diacetonitriles substituted in the 2 position by the radical R, in which R is methyl, ethyl, n-propyl or isobutyl, as intermediates.

## Revendications

1. Procédé de préparation de dérivés de l'acide glycine-N,N-diacétique de formule générale I 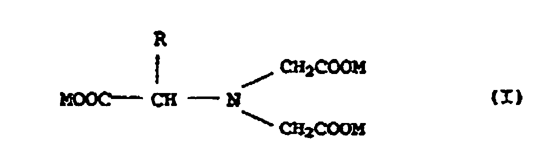 dans laquelle
R représente un groupe méthyle, éthyle, n-propyle ou isobutyle et
M représente un atome d'hydrogène, un métal alcalin, un métal alcalino-terreux, l'ammonium ou un ammonium substitué dans la quantité stoechiométrique correspondante,
**caractérisé en ce que** l'on fait réagir de l'acide iminodiacétique ou de l'iminodiacétonitrile avec les quantités correspondantes de monoaldéhydes et de cyanure d'hydrogène ou d'un cyanure d'un métal alcalin et que l'on hydrolyse ensuite les groupes résiduels de nitrile pour former des groupes carboxyle.

2. Procédé de préparation de l'acide -alanine-N,N-diacétique et de ses sels des métaux alcalins, d'ammonium et d'ammonium substitué selon la revendication 1.

3. Procédé de préparation de dérivés de l'acide glycine-N,N-diacétique I selon la revendication 1, **caractérisé en ce que** l'on travaille avec des valeurs pH allant de 0 à 11.

4. Glycine-N,N-diacétonitriles et glycinenitrile-N,N-diacétonitriles, substitués par un groupe R en postition 2, en tant que produits intermédiaires, dans lesquels le groupe R représente un groupe méthyle, éthyle, n-propyle ou isobutyle.
